# EUROPEAN PATENT APPLICATION

(11) **EP 4 257 183 A1**
(43) Date of publication of application: **11.10.2023**
(21) Application number: 21903394.1
(22) Date of filing: 07.12.2021
(51) Int. Cl.: A61P 1/00, A61P 1/12, A61P 1/14, C12N 1/20, C12N 9/88, A61K 35/741, A61K 31/715, A61K 31/734, C12P 7/40, A23L 33/125

(54) **PREBIOTIC COMPOSITION FOR BUTYRIC ACID BACTERIA**

(30) Priority: 07.12.2020 JP 2020202884; 28.05.2021 JP 2021089998
(71) Applicant: Morinaga Milk Industry Co., Ltd., Minato-ku Tokyo 108-8384 (JP)
(72) Inventor: MURAKAMI, Ryuta, Zama-shi, Kanagawa 252-8583 (JP)
(74) Representative: Hoffmann Eitle
(86) International application number: PCT/JP2021/044875
(87) International publication number: WO 2022/124295

(57) **Abstract**

An object is to provide a prebiotic which efficiently proliferates a butyrate-producing bacterium. An oligosaccharide which is composed of β-D-mannuronic acid and/or α-L-guluronic acid and which has an unsaturated form of β-D-mannuronic acid residue or α-L-guluronic acid residue at the non-reducing end or a salt thereof is used as an active ingredient of a prebiotic composition for a butyrate-producing bacterium. The oligosaccharide can be produced by causing an alginate lyase to act on alginic acid and/or a salt thereof or a hydrolysate thereof and thus obtaining a degradation product of alginic acid.

## Description

### Technical Field

The present invention relates to a prebiotic composition for a butyrate-producing bacterium.

### Background Art

In recent years, the relevance of enteric bacteria to health has been drawing attention and has been studied worldwide. The term "probiotics", which is known as a term related to the improvement of enteric environment, generally refers to living microorganisms which improve the balance of the intestinal microbiota and which thus achieve useful effects in human, and bifidobacteria and the like are generally known. Those which are assimilated by the probiotics include "prebiotics". Prebiotics generally mean those which are not degraded or absorbed in the upper gastrointestinal tract and which are selective nutrient sources for useful commensal bacteria in the large intestine, promote the growth thereof, improve and maintain the healthy balance of the intestinal microbiota composition in the large intestine and serve to improve and maintain the human health.

One of the useful commensal bacteria in the large intestine is a butyrate-producing bacterium. Butyric acid is a one of the short-chain fatty acid in the intestines and serves as the main nutrient providing energy to the large intestinal cells and also as a cell mediator which adjusts various functions not only in the intestines, such as gene expression of the host, cell differentiation, development of intestinal tissues, immunomodulation, reduction in oxidative stress and diarrhea control (NPL 1).

It is useful to ingest butyric acid for the health. However, because butyric acid emits a very strong unpleasant odor, its ingestion in the form of a food, a pharmaceutical product or the like is not practical. Moreover, the major butyrate-producing bacteria living in the intestines are extremely oxygen sensitive, and thus *in vitro* culture thereof is extremely difficult. Therefore, it is also difficult to formulate a butyrate-producing bacterium for the ingestion (NPL 2).

Accordingly, it has been proposed to promote proliferation of butyrate-producing bacteria living in the intestines, instead of ingesting butyrate-producing bacteria. For example, PTL 1 discloses that alginic acid and/or a salt thereof can promote proliferation of a butyrate-producing bacterium such as *Faecalibacterium prausnitzii* in the intestines and can be an active ingredient of a prebiotics.

### Citation List

### Patent Literature

PTL 1: WO2020/138511

### Non Patent Literature

NPL 1: Cell. 2016 Jun 2;165(6):1332-1345
NPL 2: Best. Pract. Res. Clin. Gastroenterol. 2017 Dec; 31 (6) : 643-648.

### Summary of Invention

### Technical Problem

The present inventors have examined alginic acid and/or a salt thereof as a prebiotic material for promoting a butyrate-producing bacterium and have discovered that *Faecalibacterium prausnitzii* cannot assimilate alginic acid having a certain polymerization degree or higher and/or a salt thereof and does not grow in an environment other than the intestines, namely in the absence of bacteria other than butyrate-producing bacteria.

Under the circumstances, an aspect of the invention is to provide a prebiotic which more efficiently promotes proliferation of a butyrate-producing bacterium such as *Faecalibacterium prausnitzii.*

### Solution to Problem

As a result of intensive research to achieve the object, the present inventors have found that a degradation product of alginic acid obtained by causing an alginate lyase to act on alginic acid and/or a salt thereof or a hydrolysate thereof can significantly promote proliferation of butyrate-producing bacteria including *Faecalibacterium prausnitzii* and thus have completed the invention.

That is, a first embodiment of the invention is a prebiotic composition for a butyrate-producing bacterium containing an oligosaccharide composed of β-D-mannuronic acid and/or α-L-guluronic acid or a salt thereof in which the oligosaccharide has an unsaturated form of β-D-mannuronic acid residue or α-L-guluronic acid residue at the non-reducing end.

In the embodiment, the unsaturated form preferably has a double bond between the carbon atoms at position 4 and position 5.

In the embodiment, the oligosaccharide preferably contains an oligosaccharide having a polymerization degree of 2 to 10.

In the embodiment, the butyrate-producing bacterium is preferably a bacterium of *Faecalibacterium,* more preferably *Faecalibacterium prausnitzii.*

As another aspect of the embodiment, a composition containing an oligosaccharide composed of β-D-mannuronic acid and/or α-L-guluronic acid or a salt thereof in which the oligosaccharide has an unsaturated form of β-D-mannuronic acid residue or α-L-guluronic acid residue at the non-reducing end and which is administered or inoculated to a subject having a disease or a pathological condition that can be prevented or improved through an increase in butyric acid in the body or a subject having a disease or a pathological condition that is caused by a decrease in butyric acid in the body is also provided.

The composition of the embodiment is preferably used for intestinal regulation, immunomodulation, reduction in oxidative stress, prevention or improvement of diarrhea, prevention or improvement of an inflammatory bowel disease or prevention of large intestine cancer.

The composition of the embodiment is preferably a food or a drink.

The composition of the embodiment is preferably a pharmaceutical product.

Moreover, a second embodiment of the invention is a method for producing a prebiotic composition for a butyrate-producing bacterium, including a step of causing an alginate lyase to act on alginic acid and/or a salt thereof or a hydrolysate thereof and thus obtaining a degradation product of alginic acid.

The method of the embodiment preferably includes a step of hydrolyzing the alginic acid and/or the salt thereof and thus obtaining an alginate hydrolysate and a step of causing the alginate lyase to act on the alginate hydrolysate and thus obtaining the degradation product of alginic acid.

The method of the embodiment preferably further includes a step of collecting an oligosaccharide having a polymerization degree of 2 to 3 and/or a salt thereof,

In the embodiment, the alginate lyase is preferably an endo-type alginate lyase and/or an exo-type alginate lyase.

In the embodiment, the butyrate-producing bacterium is preferably a bacterium of *Faecalibacterium,* more preferably *Faecalibacterium prausnitzii.*

### Advantageous Effects of Invention

According to the invention, a prebiotics which can more efficiently promote proliferation of *Faecalibacterium prausnitzii* is provided. Butyric acid produced by *Faecalibacterium prausnitzii* functions as a cell mediator and maintains and improves the health. Thus, the invention is industrially extremely useful.

### Brief Description of Drawings

[Fig. 1] A graph showing the turbidities of the culture solutions after 24 hours of single culture of *Faecalibacterium prausnitzii* in media to which samples were added in Test Example 1 (n=3).
[Fig. 2] A graph showing the turbidities of the media after 24 hours of single culture of *Faecalibacterium prausnitzii* in culture solutions to which samples were added in Test Example 2 (n=3).
[Fig. 3] A picture of the TLC plate in which the culture solutions before culture and after 24 hours of culture were developed in Test Example 2.
[Fig. 4] A graph showing the turbidities of the culture solutions after 24 hours of single culture of *Faecalibacterium prausnitzii* in culture solutions to which samples were added in Test Example 3 (n=3).
[Fig. 5] A picture of the TLC plate in which the culture solutions before culture and after 24 hours of culture were developed in Test Example 3.
[Fig. 6] A picture of the TLC plate in which samples obtained by hydrolysis treatment and/or lyase degradation treatment of sodium alginate were developed in Test Example 4.
[Fig. 7] A graph showing the turbidities of the culture solutions after 24 hours of single culture of *Faecalibacterium prausnitzii* in culture solutions to which samples were added in Test Example 5 (n=3).
[Fig. 8] A picture of the TLC plate in which samples obtained by hydrolysis treatment and then lyase degradation treatment of sodium alginate were developed in Test Example 6.
[Fig. 9] A graph showing the oligosaccharide amounts contained in samples of a lyase-treated product of potassium alginate before and after permeation through an NF membrane for respective polymerization degrees in Test Example 7. The peak areas in a HPLC chart of the samples were used as the oligosaccharide amounts having the corresponding polymerization degrees.
[Fig. 10] A picture of the TLC plate in which samples of a lyase-treated product of potassium alginate before and after permeation through an NF membrane were developed in Test Example 7.
[Fig. 11] A graph showing the change with time of the proportion of *Faecalibacterium prausnitzii* in all the enteric bacteria after neutralizing culture using an NF membrane permeate of a lyase-treated product of potassium alginate in Test Example 8 (n=4).
[Fig. 12] A graph showing the turbidity of the culture solution after 24 hours of single culture of *Faecalibacterium prausnitzii* in a culture solution to which an NF membrane permeate of a lyase-treated product of potassium alginate was added in Test Example 9 (n=3).

### Description of Embodiments

Next, the invention is explained in detail. However, the invention is not limited to the following embodiments and can be changed freely within the scope of the invention.

In the present specification, a numerical range expressed with "to" means a numerical range including the values before and after the "to" as the lower limit and the upper limit, unless otherwise specified.

The composition of the invention contains an oligosaccharide composed of β-D-mannuronic acid and/or α-L-guluronic acid or a salt thereof. The oligosaccharide generally has a linear chain in which pyranose rings are linked with a β1→4 glycoside bond but is not particularly limited. The saccharides constituting the oligosaccharide may be all β-D-mannuronic acid, all α-L-guluronic acid or β-D-mannuronic acid and α-L-guluronic acid in any order at any ratio.

The composition of the invention may contain an oligosaccharide other than the oligosaccharide or the salt thereof according to the invention.

The oligosaccharide according to the invention has an unsaturated form of β-D-mannuronic acid residue or α-L-guluronic acid residue at the non-reducing end. The unsaturated form generally has a double bond between the carbon atoms at position 4 and position 5 of the non-reducing end saccharide.

As shown in the Examples below, compared to an alginate hydrolysate which has not been treated with a lyase, namely an oligosaccharide composed of β-D-mannuronic acid and/or α-L-guluronic acid without any unsaturated end, the oligosaccharide according to the invention is more easily assimilated by a butyrate-producing bacterium, and thus it is speculated that the assimilation property of a butyrate-producing bacterium is improved when the oligosaccharide has an unsaturated form at the non-reducing end.

The polymerization degree of the oligosaccharide according to the invention is preferably 2 to 10, more preferably 2 to 6, further preferably 2 to 5, particularly preferably 2 to 3. Here, the oligosaccharide in the composition of the invention preferably contains an oligosaccharide having a polymerization degree of 2 to 10, but this does not prevent an oligosaccharide having a polymerization degree of 11 or more from being contained.

As shown in the Examples below, an oligosaccharide having a low polymerization degree, especially an oligosaccharide having a polymerization degree of 2 to 3, is easily assimilated by a butyrate-producing bacterium. Accordingly, an oligosaccharide having a polymerization degree of 2 to 3 and/or a salt thereof is contained, based on the entire oligosaccharide or the salt thereof contained in the composition of the invention, preferably at 30 mass% or more, more preferably at 50 mass% or more, further preferably at 90 mass% or more.

The salt of the oligosaccharide is a sodium salt, a potassium salt, a calcium salt, an ammonium salt or the like. The salt is not particularly limited in the invention, but a sodium salt and a potassium salt are preferable in view of the solubility in water.

A commercial oligosaccharide can be used as the oligosaccharide according to the invention. For example, oligosaccharides are sold by Hokkaido Mitsui Chemicals, Inc., Shandong, Runxin and the like under the name of "alginate oligosaccharides (AOSs)".

The oligosaccharide according to the invention can also be obtained by enzymatically degrading alginic acid and/or a salt thereof or a hydrolysate thereof with a lyase.

That is, the present specification discloses a method for producing a prebiotic composition for a butyrate-producing bacterium, including a step of causing an alginate lyase to act on alginic acid and/or a salt thereof or a hydrolysate thereof and thus obtaining a degradation product of alginic acid.

Alginic acid is a polysaccharide contained in marine algae such as tangle weed and *Undaria pinnatifida* and is widely used, including for the application to foods as a thickening and stabilizing agent. Alginic acid has a linear structure composed of β-D-mannuronic acid and α-L-guluronic acid linked with a β1-4 bond. The polymerization degree of alginic acid varies with the origin, but the molecular weight is generally about 40,000 to several millions.

The salt of alginic acid is a sodium salt, a potassium salt, a calcium salt, an ammonium salt or the like. The salt is not particularly limited in the invention, but a sodium salt and a potassium salt are preferable in view of the solubility in water.

The alginic acid and/or the salt thereof which are used for the production method of the invention can be obtained by extracting from a marine alga or the like, and as commercial products, for example, "I-S (molecular weight of about three million to four million)", "I-5 (molecular weight of about 2.8 million)", "ULV-L3 (molecular weight of 40,000 to 60,000)" and "IL-6 (molecular weight of about 40,000 to 60,000)" (all manufactured by KIMICA Corporation) and the like can be acquired and used.

As described above, because an oligosaccharide having a low polymerization degree is more easily assimilated by a butyrate-producing bacterium, when the oligosaccharide according to the invention is produced from alginic acid and/or a salt thereof as a raw material, the alginic acid and/or the salt thereof is preferably hydrolyzed before degradation treatment with a lyase to obtain an oligosaccharide having a low polymerization degree.

In a preferable embodiment, the oligosaccharide according to the invention is produced by a step of causing an alginate lyase to act on a hydrolysate of alginic acid and/or a salt thereof and thus obtaining a degradation product of alginic acid. Moreover, in a preferable embodiment, the oligosaccharide according to the invention is produced by a step of hydrolyzing alginic acid and/or a salt thereof and thus obtaining an alginate hydrolysate and a step of causing an alginate lyase to act on the alginate hydrolysate and thus obtaining a degradation product of alginic acid.

Because the optimum temperature of the enzymatic reaction of an alginate lyase is around 40 degrees, microorganisms which are not preferable for foods easily grow during the enzymatic reaction. By the embodiment including a step of causing an alginate lyase to act on a hydrolysate of alginic acid and/or a salt thereof and thus obtaining a degradation product of alginic acid, or the embodiment including a step of hydrolyzing alginic acid and/or a salt thereof and thus obtaining an alginate hydrolysate, and a step of causing an alginate lyase to act on the alginate hydrolysate and thus obtaining a degradation product of alginic acid, the time required to cause the alginate lyase to act can be shortened, and the growth of the microorganisms can be suppressed.

The hydrolysis treatment is not prevented from being conducted after the degradation treatment with the lyase but is preferably conducted before the degradation treatment with the lyase.

This is to increase the proportion of the oligosaccharide according to the invention, namely an oligosaccharide having an unsaturated form of β-D-mannuronic acid residue or α-L-guluronic acid residue at the non-reducing end, in the entire oligosaccharide obtained. As shown in the Examples below, an oligosaccharide obtained by first hydrolyzing sodium alginate and then degrading with a lyase is more easily assimilated by *Faecalibacterium prausnitzii* and can promote the growth thereof.

The hydrolysate of alginic acid and/or a salt thereof which is subjected to the treatment with an alginate lyase may also be an oligosaccharide having a polymerization degree of preferably 20 or less, more preferably 10 or less, further preferably 8 or less, particularly preferably 5 or less. Alternatively, the weight-average molecular weight which is measured by a HPLC method conducted under the following conditions may be preferably 10000 or less, more preferably 8000 or less, further preferably 7000 or less, particularly preferably 5000 or less.
Measurement Device: Ultimate 3000 (manufactured by Thermo)
Detection: Refracto Max 521 detector (manufactured by Thermo)
   Column: TSKgel G3000PW (manufactured by Tosoh Corporation)
Mobile Phase: 0.1M aqueous sodium nitrate solution
Flow Rate: 0.3 mL/min
Column Temperature: 40°C
Standard: STANDARD P-82 (manufactured by Shodex)

The molecular weight measured by the HPLC conducted under the above conditions is an "apparent" value which may be different from the "true" molecular weight calculated from the actual polymerization degree. When an alginate oligosaccharide having a polymerization degree of 4 to 5 is measured under the above conditions, the molecular weight is generally about 3500.

The method for hydrolyzing alginic acid and/or a salt thereof is preferably an acid hydrolysis method but is not particularly limited.

The acid hydrolysis is conducted specifically by adjusting the pH of an aqueous solution of alginic acid and/or a salt thereof to around 3 to 5 and heating at a high temperature of, for example, 100°C or higher, for several hours, and the adjustment of the pH is conducted by adding a weak acid such as acetic acid but is not limited thereto. For example, when a 0.3 v/v% aqueous acetic acid solution containing 3 mass% commercial sodium alginate having a molecular weight of 40,000 to 60,000 is subjected to acid hydrolysis through autoclave treatment at 104°C for 7.5 hours, a hydrolysate having a weight-average molecular weight of about 6000 can be obtained.

As described above, the method for producing the oligosaccharide according to the invention includes a step of causing an alginate lyase to act on alginic acid and/or a salt thereof or a hydrolysate thereof and thus obtaining a degradation product of alginic acid.

The alginate lyase cleaves the β1→4 glycoside bond of alginic acid and forms a double bond between the carbon atoms at position 4 and position 5 of the β-D-mannuronic acid residue or the α-L-guluronic acid residue at the non-reducing end of the degradation product. Accordingly, the degradation product of alginic acid (also called a lyase-treated alginic acid product) in the production method of the invention can be the oligosaccharide according to the composition of the invention.

The ratio of the β-D-mannuronic acid residues and α-L-guluronic acid residues in the oligosaccharide or the degradation product of alginic acid produced by the production method of the invention depends on the origin of alginic acid as the raw material.

As the alginate lyase in the production method of the invention, any of an endo-type alginate lyase, an exo-type alginate lyase, or a mixture thereof may be used.

As the enzyme, a commercial enzyme can be obtained. Examples thereof include "alginate lyase S" (manufactured by Nagase ChemteX Corporation), "HULK alginate lyase" (manufactured by Nippon Gene Co., Ltd.) and the like, and a kind or two or more kinds of the enzymes may be selected and used.

The amount of the alginate lyase used for alginic acid and/or a salt thereof or a hydrolysate thereof is not particularly limited and may be appropriately adjusted depending on the substrate concentration, the enzyme potency, the reaction temperature, the reaction period and the like. However, the alginate lyase is generally preferably added at a ratio of 20 to 100 units per 1 g of the alginic acid and/or the salt thereof or the hydrolysate thereof.

The pH of the enzymatic reaction system may be adjusted to the optimum pH of the used enzyme using a salt which can be used for foods, such as potassium carbonate and sodium hydroxide. For example, the pH of the reaction solution is adjusted to preferably 5 to 8, more preferably 6 to 7.

The reaction temperature of the alginate lyase is preferably in the optimum temperature range of the used enzyme and is preferably 30 to 50°C, more preferably 40 to 45°C.

The reaction period of the alginate lyase may be appropriately adjusted, and the reaction can be conducted, for example, for 0.5 to 24 hours, preferably for 0.5 to 12 hours, more preferably for 0.5 to 6 hours.

The degradation reaction by the alginate lyase may be terminated by inactivating the enzyme by heating. For example, the inactivation is suitably conducted for one to three seconds when the enzyme is inactivated at 100°C or higher (suitably at 110 to 130°C) and for three to 40 minutes when the enzyme is inactivated at 60°C or higher and lower than 100°C.

After the completion of the enzymatic degradation, the pH of the solution of the degradation product of alginic acid may be adjusted preferably to around 6 to 8 according to the need.

In the production method of the invention, the degradation product of alginic acid obtained by the alginate lyase reaction contains an oligosaccharide having a polymerization degree of 2 to 10 at preferably 30 mass% or more, more preferably 50 mass% or more, further preferably 90 mass% or more of the entire degradation product. The degradation reaction by the alginate lyase is preferably advanced to achieve the range, and accordingly, the reaction conditions are preferably adjusted appropriately.

The degradation product of alginic acid after the enzymatic reaction may be used in the unpurified state but may be further appropriately subjected to known separation and purification. For example, by subjecting the obtained degradation product of alginic acid to membrane separation, molecular weight fractionation, ethanol precipitation or the like, a fraction of an oligosaccharide having an appropriate polymerization degree or molecular weight can be obtained.

A preferable embodiment of the production method of the invention further includes a step of collecting an oligosaccharide having a polymerization degree of 2 to 3 and/or a salt thereof. In the production method of the invention, timing for conducting the collection step is not particularly limited but is generally after the degradation step by the alginate lyase.

By the collection step, the proportion of an oligosaccharide having a polymerization degree of 2 to 3 and/or a salt thereof in the entire oligosaccharide should be increased compared to that before the collection step, and an oligosaccharide having a polymerization degree of 4 or more is not prevented from being contained in the oligosaccharide after the collection step.

As the membrane separation which can be used for the collection step, a method of permeating through an NF (nanofiltration) membrane, an RO (reverse osmosis) membrane or the like can be employed, and an oligosaccharide having a polymerization degree of 2 to 3 and/or a salt thereof can be collected as the permeate of the membrane. The conditions for the membrane separation treatment (the pressure, the flow rate or the like) are not particularly limited as long as desired collection is possible and may be appropriately adjusted.

As the molecular weight fractionation which can be used for the collection step, for example, a method such as HPLC can be employed, and oligosaccharides having unnecessary molecular weights and undegraded alginic acid can be thus removed.

Furthermore, a known separation and purification method (for example, an ion exchange resin or the like) may also be used to remove a salt or impurities or to increase the purity.

The amount of the oligosaccharide and/or the salt thereof described above in the composition of the invention may be appropriately set depending on the embodiment of the composition and is not particularly limited but is, for example, preferably 0.1 mass% or more of the entire composition, more preferably 1 mass% or more, further preferably 10 mass% or more. The upper limit of the amount is not particularly restricted but may be, for example, 100 mass% or less of the entire composition, more preferably 80 mass% or less, further preferably 50 mass% or less. In the case of an oligosaccharide salt, these values are values in terms of the oligosaccharide. These amounts may be any of the values during the production of the composition of the invention, the values during the distribution and the values for the intake (administration).

The composition of the invention is used as a prebiotic for a butyrate-producing bacterium. That is, the composition is assimilated by a butyrate-producing bacterium and can promote the growth thereof. The promotion here refers to an increase in the amount or the growth rate of a butyrate-producing bacterium compared to that without the intake of the oligosaccharide according to the invention.

Butyrate-producing bacterium is a generic term for the bacteria which produce butyric acid. The butyrate-producing bacterium in the invention is not particularly limited. However, examples thereof include those of *Faecalibacterium,* and a more specific example is *Faecalibacterium prausnitzii* in the human intestines or the like.

The composition of the invention can be useful for a subject having a disease or a pathological condition which can be prevented or improved through an increase in butyric acid in the body or a disease or a pathological condition which is caused by a decrease in butyric acid in the body. For example, the composition can be for intestinal regulation, for immunomodulation, for reduction in oxidative stress, for prevention/improvement of diarrhea, for an inflammatory bowel disease, for prevention of large intestine cancer or the like.

Another embodiment of the invention is use of an oligosaccharide which is composed of β-D-mannuronic acid and/or α-L-guluronic acid and which has an unsaturated form of β-D-mannuronic acid residue or α-L-guluronic acid residue at the non-reducing end or a salt thereof in the manufacture of a prebiotic composition for a butyrate-producing bacterium.

Another embodiment of the invention is use of an oligosaccharide which is composed of β-D-mannuronic acid and/or α-L-guluronic acid and which has an unsaturated form of β-D-mannuronic acid residue or α-L-guluronic acid residue at the non-reducing end or a salt thereof in the promotion of the growth of a butyrate-producing bacterium.

Another embodiment of the invention is an oligosaccharide which is composed of β-D-mannuronic acid and/or α-L-guluronic acid and which has an unsaturated form of β-D-mannuronic acid residue or α-L-guluronic acid residue at the non-reducing end or a salt thereof for use in the promotion of the growth of a butyrate-producing bacterium.

It is a method for promoting the growth of a butyrate-producing bacterium including administering an oligosaccharide which is composed of β-D-mannuronic acid and/or α-L-guluronic acid and which has an unsaturated form of β-D-mannuronic acid residue or α-L-guluronic acid residue at the non-reducing end or a salt thereof to an animal. The animal here is not particularly limited but is generally a human.

The timing of intake (administration) of the composition of the invention is not particularly limited and can be appropriately selected depending on the condition of the subject of the administration.

The intake (dosage) of the composition of the invention is appropriately selected based on the age of the subject of the intake (administration), the gender, the condition, other conditions and the like. A standard amount of the oligosaccharide according to the invention is an amount falling in the range of preferably 1 to 300 mg/kg/day, more preferably 20 to 50 mg/kg/day.

Regardless of the amount or the period of intake (administration), the composition can be administered once a day or in multiple divided portions.

The route of intake (administration) of the composition of the invention may be an oral or parenteral route but is generally an oral route. The parenteral intake (administration) is rectal administration or the like.

In an embodiment, the composition of the invention may contain a butyrate-producing bacterium with the oligosaccharide according to the invention. The composition of the invention may be taken in combination with a butyrate-producing bacterium or with a preparation containing a butyrate-producing bacterium. By the embodiment of administering the composition, an effect of promoting the growth of the butyrate-producing bacterium in the intestine and an effect of thus increasing butyric acid are expected. In these embodiments, the butyrate-producing bacterium is preferably a living bacterium.

When the composition of the invention is an orally taken composition, the composition is preferably a food or a drink in an embodiment.

The form and the property of the food or the drink are not particularly restricted as long as the food or the drink does not impair the effects of the invention and can be orally taken, and the food or the drink can be produced by a general method using a material which is generally used for a food or a drink except that the oligosaccharide or the salt thereof according to the invention is added to the material.

A prebiotic food or drink for a butyrate-producing bacterium can also be produced by a method including a step of adding an oligosaccharide and/or a salt thereof generally obtained by the production method of the invention as described above to a food or drink material.

The food or the drink is not limited regarding the form such as liquid, paste, gel solid or powder. Examples include the following examples: tablet candies; liquid foods (nutrition products for tube feeding); wheat products such as breads, macaroni, spaghetti, noodles, cake mixes, frying flours and bread crumbs; instant foods such as instant noodles, cup noodles, retort-pouched/prepared foods, prepared canned foods, microwave foods, instant soups/stews, instant miso soups/clear Japanese soups, canned soups, freeze-dried foods and other instant foods; processed agricultural products such as canned agricultural products, canned fruits, jams/marmalades, pickles, cooked beans, dried agricultural products and cereals (processed grains); processed fishery products such as canned fishery products, fish hams/sausages, fishery paste products, fishery delicacies and *Tsukudani* (foods boiled down in sweetened soy sauce); processed livestock products such as canned livestock products/pastes and livestock hams/sausages; milk/dairy products such as processed milk, milk beverages, yogurts, lactic acid bacteria beverages, cheeses, ice creams, creams and other dairy products; oils and fats such as butter, margarine and vegetable oils; basic condiments such as soy sauce, soybean paste, sauces, processed tomato condiments, *Mirin* (sweet sake for seasoning) and vinegars; compound flavor enhancers/foods such as cooking mixes, curry roux, sauces, dressings, noodle broths, spices and other compound flavor enhancers; frozen foods such as frozen food materials, semi-cooked frozen foods and cooked frozen foods; confectioneries such as caramels, candies, chewing gums, chocolates, cookies, biscuits, cakes, pies, snacks, crackers, Japanese-style confectioneries, rice confectioneries, bean confectioneries, desserts, jellies and other confectioneries; luxury beverages such as carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols and other luxury beverages, other commercial foods such as baby foods, *Furikake* (dry Japanese seasonings) and seasonings for *Chazuke* (boiled rice with hot tea) and the like; formula for infants and the like (including powdered formula, liquid formula and the like); enteral nutrition products; functional foods (foods for specified health uses, foods with nutrient function claims or foods with function claims) and nutritional supplements; and the like.

An embodiment of the food or the drink can be feed. The feed is pet food, livestock feed, fish farming feed or the like.

The form of the feed is not particularly restricted and may contain, in addition to the oligosaccharide or the salt thereof according to the invention, for example: alginic acid or a salt thereof; an oligosaccharide other than the oligosaccharide according to the invention; grain such as corn, wheat, barley, rye and milo; vegetable oil cake such as soybean oil cake, rapeseed oil cake, coconut oil cake and linseed oil cake; bran such as oat bran, wheat bran, rice bran and defatted rice bran; a food manufacturer's by-product such as corn gluten meal and corn jam meal; animal feed such as fish powder, defatted milk powder, whey, yellow grease and tallow; yeast such as torula yeast and brewer's yeast; mineral feed such as tertiary calcium phosphate and calcium carbonate; an oil or a fat; a single amino acid; a saccharide; or the like.

When the composition of the invention is an embodiment of a food or a drink (including feed), the composition can be provided/sold as a food or a drink labeled for use as a prebiotic for a butyrate-producing bacterium or for promoting the growth of a butyrate-producing bacterium in an intestine.

The "labeling" act includes all the acts for informing a consumer of the use, and all the expressions which can remind of/cause to guess the use are the "labeling" acts of the invention, regardless of the purposes of labeling, the contents of labeling, the objects to be labeled, the media and the like.

The "label" preferably contains an expression which allows a consumer to directly recognize the use. Specific examples include an act of transferring an article in which the use is described on a product regarding the food or the drink or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (internet or the like) and another act.

The content of the label is preferably a label approved by the administration or the like (for example, a label approved based on a system provided by the administration and provided in the form based on the approval or the like). It is preferable to affix the label with such a content on packaging, a container, a catalogue, a brochure, an advertisement material in a sales site such as POP, other documents or the like.

The "labels" also include labels with health foods, functional foods, enteral nutrition products, food for special dietary uses, food with health claims (foods for specified health uses, foods with nutrient function claims and foods with function claims), nutritional supplements, quasi-drugs and the like. In particular, the labels are labels approved by the Consumer Affairs Agency, such as labels approved by the systems for foods for specified health uses, foods with nutrient function claims or foods with function claims or by a similar system and the like. Specific examples include a label with foods for specified health uses, a label with qualified foods for specified health uses, a label indicating influence on the structure or the function of a body, a label with reduction of disease risk, a label with a scientifically grounded function and the like. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The labels are, for example, labels with "for those who wish to increase butyrate-producing bacteria in the stomach", "for those who wish to increase bacteria of *Faecalibacterium",* "intestinal regulation effect with butyric acid", "improving the immunity by increasing butyrate-producing bacteria" and the like.

In an embodiment, the composition of the invention can be a pharmaceutical product.

The administration route of the pharmaceutical product may be an oral or parenteral route but is preferably an oral route. The parenteral intake (administration) is rectal administration or the like.

Regarding the form of the pharmaceutical product, the composition can be appropriately formulated into a desired dosage form depending on the administration method. For example, in the case of oral administration, the composition can be formulated into a solid preparation such as powder, granules, tablets and capsules, a liquid preparation such as a solution, a syrup, a suspension and an emulsion or the like. In the case of parenteral administration, the composition can be formulated into a suppository, ointment, an injection or the like.

For the formulation, in addition to the oligosaccharide or the salt thereof according to the invention, a component which is generally used for formulation such as excipients, pH-adjusting agents, colorants and corrigents can be used. Another medicinal component, a prebiotic which is known or will be found in the future or the like can also be used in combination.

In addition, the formulation can be appropriately conducted by a known method depending on the dosage form. For the formulation, a carrier for formulation can be appropriately blended and formulated.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of binders include, in addition to the excipients: gelatin; polyvinylpyrrolidone; macrogol; and the like.

Examples of disintegrating agents include, in addition to the excipients, chemically modified starch or cellulose derivatives such as croscarmellose sodium, sodium carboxymethyl starch and cross-linked polyvinylpyrrolidone and the like.

Examples of lubricants include: talc; stearic acid; metal stearates such as calcium stearate and magnesium stearate; colloidal silica; waxes such as Veegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of flavoring agents include sweeteners, acidulants, aromas and the like.

In this regard, the carriers used in the case of a liquid preparation for oral administration include solvents such as water and the like.

The timing for taking the pharmaceutical product of the invention is not particularly limited, and examples include before a meal, after a meal, between meals, before bedtime and the like.

### Examples

The invention is explained further specifically below using Examples, but the invention is not limited to these Examples.

In Test Example 2 and later, the same materials and the same reagents as those used in Test Example 1 were used unless otherwise specified.

### <Test Example 1> Single Culture 1 of Faecalibacterium prausnitzii

### (1) Medium Preparation

To 99.7 mL of MilliQ water, 0.3 mL of acetic acid (Kokusan Chemical Co., Ltd.) was added, and the mixture was subjected to autoclave treatment at 104°C for 7.5 hours. Then, the obtained sterilized water was subjected to SpeedVac to completely remove acetic acid and water, and 40 mL of MilliQ water was added thereto. The mixture was subjected to SpeedVac again to completely remove water. By adding 64.7 mL of MilliQ water again, an aqueous solution for medium preparation was obtained.

To the total amount of the aqueous solution for medium preparation prepared above, 1 g of a commercial lyase-treated alginic acid product 1 ("alginate oligosaccharide" (manufactured by Hokkaido Mitsui Chemicals, Inc.)) was added, and a specified amount of powder having YCFA medium composition excluding the saccharides was further added. A medium containing the lyase-treated alginic acid product 1 was thus obtained.

To the total amount of the aqueous solution for medium preparation prepared above, 1 g of a commercial sodium alginate material ("ULV-L3 (molecular weight of 40,000 to 60,000)" (manufactured by KIMICA Corporation)) was added, and a specified amount of powder having YCFA medium composition excluding the saccharides was further added. The mixture was subjected to autoclave treatment at 115°C for 15 minutes, and a sodium alginate-containing medium was thus obtained.

A commercial sodium alginate material ("ULV-L3 (molecular weight of 40,000 to 60,000)" (manufactured by KIMICA Corporation)) in an amount of 1 g was dissolved with 99.7 mL of MilliQ water, and 0.3 mL of acetic acid was added. The mixture was subjected to autoclave treatment at 104°C for 7.5 hours. Then, the mixture was subjected to SpeedVac to completely remove acetic acid and water, and 40 mL of MilliQ water was added thereto. The mixture was subjected again to SpeedVac to completely remove water. By adding 64.7 mL of MilliQ water again, an aqueous solution of an alginate hydrolysate was obtained. A specified amount of powder having YCFA medium composition excluding the saccharides was added thereto, and the mixture was subjected to autoclave treatment at 115°C for 15 minutes. A medium containing an alginate hydrolysate was thus obtained.

To the total amount of the aqueous solution for medium preparation prepared above, 0.2 g of glucose, 0.2 g of maltose and 0.2 g of cellobiose (all manufactured by Nacalai Tesque, Inc.) were added, and a specified amount of powder having YCFA medium composition excluding the saccharides was further added. The mixture was subjected to autoclave treatment at 115°C for 15 minutes, and a positive control medium was thus obtained.

A specified amount of powder having YCFA medium composition excluding the saccharides was added to the total amount of the aqueous solution for medium preparation prepared above. The mixture was subjected to autoclave treatment at 115°C for 15 minutes, and a negative control medium was thus obtained.

### (2) Single Culture

A vitamin solution and a cysteine solution which were sterilized by filtration were aseptically added to the YCFA media prepared in (1), and culture solutions containing the saccharides to be tested at a final concentration of 1% (w/v) were thus prepared. The culture solutions each in a volume of 2 mL were dispensed to 5-mL tubes 352063 (manufactured by Falcon). Then, the 5-mL tubes were left still in a Coy anaerobic chamber (manufactured by Coy) overnight, and the culture solutions were thus brought to anaerobic conditions. *Faecalibacterium prausnitzii* MCC2041 (deposited as an international deposit on September 20, 2019 to NITE Patent Microorganisms Depositary, National Institute of Technology and Evaluation (#122, 2-5-8 Kazusakamatari, Kisarazu-shi, Chiba 292-0818) under an accession number of NITE BP-03027) was inoculated thereto 24 hours before subjecting to the culture experiment and anaerobically cultured at 37°C, and 60 µL of thus obtained pre-culture solutions were inoculated to the culture solutions and anaerobically cultured for 24 hours at 37°C.

The turbidities at a wavelength of 600 nm of the culture solutions were measured using a downward fluorescence analyzer (manufactured by Hitachi High-Tech Science Corporation, SH9000-Lab).

### (3) Results

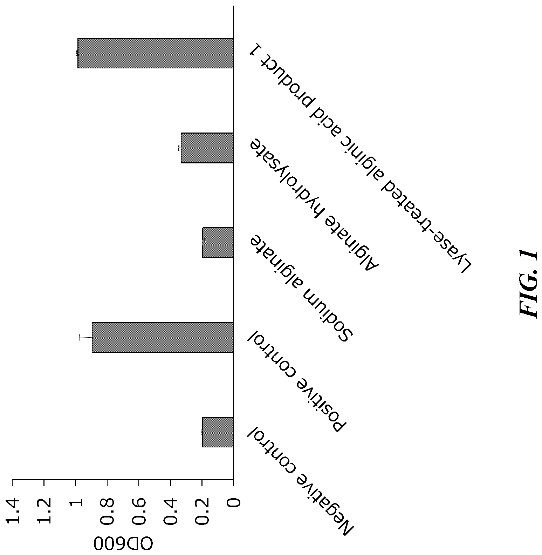

The turbidities (OD₆₀₀) of the culture solutions after 24 hours of the culture are shown in Fig. 1. *Faecalibacterium prausnitzii* could not assimilate sodium alginate and hardly grew. However, assimilation of the alginate hydrolysate was observed, and significant growth was observed in the lyase-treated alginic acid product 1.

### <Test Example 2> Single Culture 2 of Faecalibacterium prausnitzii

### (1) Medium Preparation

The lyase-treated alginic acid product 1 used in Test Example 1, a lyase-treated alginic acid product 2 ("alginate oligosaccharide" (manufactured by Shandong)) or a lyase-treated alginic acid product 3 ("alginate oligosaccharide" (manufactured by Runxin)) each in an amount of 1 g was added to 64.7 mL of MilliQ water, and a specified amount of powder having YCFA medium composition excluding the saccharides was further added. The mixtures were subjected to autoclave treatment at 115°C for 15 minutes, and media containing the lyase-treated alginic acid products 1 to 3 were thus obtained.

A commercial sodium alginate material in an amount of 1 g was added to 64.7 mL of MilliQ water, and a specified amount of powder having YCFA medium composition excluding the saccharides was further added. The mixture was subjected to autoclave treatment at 115°C for 15 minutes, and a sodium alginate-containing medium was thus obtained.

A commercial guar gum hydrolysate ("Sunfiber" (manufactured by Taiyo Kagaku Corporation)) in an amount of 1 g was added to 64.7 mL of MilliQ water, and a specified amount of powder having YCFA medium composition excluding the saccharides was further added. The mixture was subjected to autoclave treatment at 115°C for 15 minutes, and a guar gum hydrolysate-containing medium was thus obtained. Here, the guar gum hydrolysate is already known to have a capability of proliferating *Faecalibacterium prausnitzii* in the intestines.

To 64.7 mL of MilliQ water, 0.2 g of glucose, 0.2 g of maltose and 0.2 g of cellobiose (all manufactured by Nacalai Tesque, Inc.) were added, and a specified amount of powder having YCFA medium composition excluding the saccharides was further added. The mixture was subjected to autoclave treatment at 115°C for 15 minutes, and a positive control medium was thus obtained.

A specified amount of powder having YCFA medium composition excluding the saccharides was added to 64.7 mL of MilliQ water. The mixture was subjected to autoclave treatment at 115°C for 15 minutes, and a negative control medium was thus obtained.

### (2) Single Culture

*Faecalibacterium prausnitzii* MCC2041 was cultured using the YCFA media prepared in (1) in the same manner as that in Test Example 1.

### (3) Analysis of Faecalibacterium prausnitzii-Assimilated Fractions

The polymerization degrees of the oligosaccharides assimilated by *Faecalibacterium prausnitzii* were analyzed by thin-layer chromatography (TLC).

The culture solutions before the culture and after 24 hours of the culture in (2) were each centrifuged at 13000×g at 4°C for three minutes, and the supernatants were collected and used as samples.

The samples each in a volume of 1 µL were spotted on an aluminum plate silica gel 60 for thin-layer chromatography (manufactured by Merck) and developed using a development solvent (formic acid:1-butanol:distilled water = 6:4:1). After spraying a diphenylamine-aniline-phosphoric acid reagent (100 mL of acetone, 1 g of diphenylamine, 1 mL of aniline and 10 mL of phosphoric acid), the saccharides were colored by heating.

### (4) Results

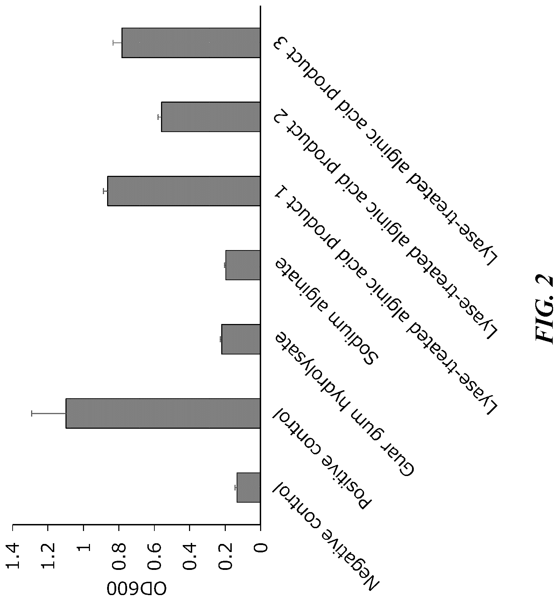

The turbidities (OD₆₀₀) of the culture solutions after 24 hours of the culture are shown in Fig. 2. *Faecalibacterium prausnitzii* could not assimilate sodium alginate and the guar gum hydrolysate and hardly grew. On the other hand, in all of the lyase-treated alginic acid products 1 to 3, significant growth of *Faecalibacterium prausnitzii* was observed.

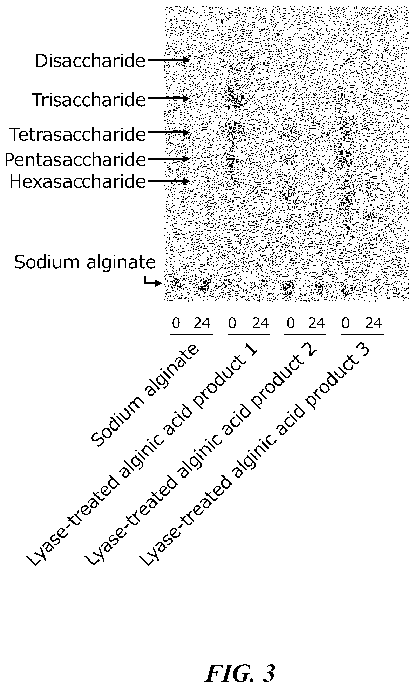

The TLC plate after coloration is shown in Fig. 3. It was observed that the spots corresponding to oligosaccharides having a polymerization degree of 2 to 6 became lighter or disappeared after the culture in the media containing the lyase-treated alginic acid products 1 to 3. This shows that *Faecalibacterium prausnitzii* assimilated especially oligosaccharides having a polymerization degree of 2 to 6 of the lyase-treated alginic acid products.

### <Test Example 3> Single Culture 3 of Faecalibacterium prausnitzii

### (1) Medium Preparation

A commercial sodium alginate material in an amount of 5 g was dissolved with 96 mL of MilliQ water. An aqueous solution of 5 mg/mL alginate lyase S (manufactured by Nagase ChemteX Corporation) in a volume of 4 mL was added thereto, and enzymatic reaction was advanced at 40°C for 24 hours. Next, the enzyme was inactivated by heating at 80°C for 60 minutes. The reaction solution was centrifuged at 8000×g for 20 minutes, and the supernatant was collected. A 5% aqueous solution of a lyase-treated alginic acid product 4 was thus obtained. The aqueous solution of the lyase-treated alginic acid product 4 in a volume of 20 mL and 2.24 times the volume of MilliQ water were mixed, and a specified amount of powder having YCFA medium composition excluding the saccharides was further added. The mixture was subjected to autoclave treatment at 115°C for 15 minutes, and a medium containing the lyase-treated alginic acid product 4 was thus obtained.

A 0.02 mass% aqueous solution of alginate lyase S (manufactured by Nagase ChemteX Corporation) was incubated at 40°C for 24 hours, and then the enzyme was inactivated by heating at 80°C for 60 minutes. The aqueous solution was centrifuged at 8000×g for 20 minutes, and the supernatant was collected. The supernatant in a volume of 20 mL and 2.24 times the volume of MilliQ water were mixed, and the mixture was subjected to autoclave treatment at 115°C for 15 minutes. An aqueous solution for medium preparation was thus obtained.

To the total amount of the aqueous solution for medium preparation prepared above, 1 g of a sodium alginate material was added, and a specified amount of powder having YCFA medium composition excluding the saccharides was further added. The mixture was subjected to autoclave treatment at 115°C for 15 minutes, and a sodium alginate-containing medium was thus obtained.

To the total amount of the aqueous solution for medium preparation prepared above, 1 g of a commercial guar gum hydrolysate ("Sunfiber" (manufactured by Taiyo Kagaku Corporation)) was added, and a specified amount of powder having YCFA medium composition excluding the saccharides was further added. The mixture was subjected to autoclave treatment at 115°C for 15 minutes, and a guar gum hydrolysate-containing medium was thus obtained.

To the total amount of the aqueous solution for medium preparation prepared above, 0.2 g of glucose, 0.2 g of maltose and 0.2 g of cellobiose were added, and a specified amount of powder having YCFA medium composition excluding the saccharides was further added. The mixture was subjected to autoclave treatment at 115°C for 15 minutes, and a positive control medium was thus obtained.

A specified amount of powder having YCFA medium composition excluding the saccharides was added to the total amount of the aqueous solution for medium preparation prepared above. The mixture was subjected to autoclave treatment at 115°C for 15 minutes, and a negative control medium was thus obtained.

### (2) Single Culture

*Faecalibacterium prausnitzii* MCC2041 was cultured using the YCFA media prepared in (1) in the same manner as that in Test Example 1.

The turbidities at a wavelength of 600 nm of the culture solutions were measured in the same manner as that in Test Example 1.

### (3) Analysis of Faecalibacterium prausnitzii-Assimilated Fractions

The polymerization degrees of the oligosaccharides assimilated by *Faecalibacterium prausnitzii* were analyzed by TLC in the same manner as that in Test Example 2.

### (4) Results

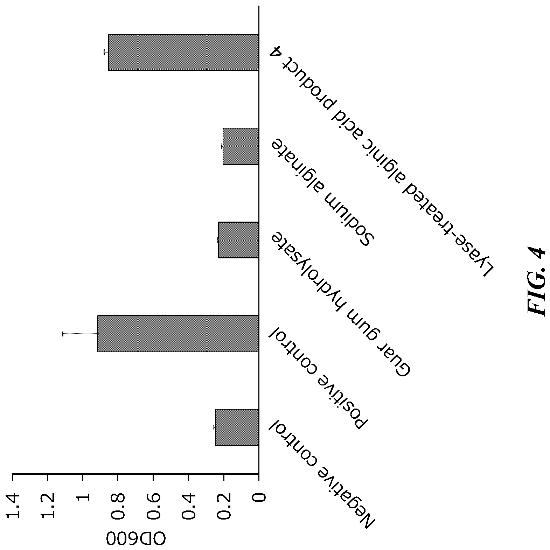

The turbidities (OD₆₀₀) of the culture solutions after 24 hours of the culture are shown in Fig. 4. *Faecalibacterium prausnitzii* could not assimilate sodium alginate and the guar gum hydrolysate and hardly grew. On the other hand, in the lyase-treated alginic acid product 4, significant growth of *Faecalibacterium prausnitzii* was observed.

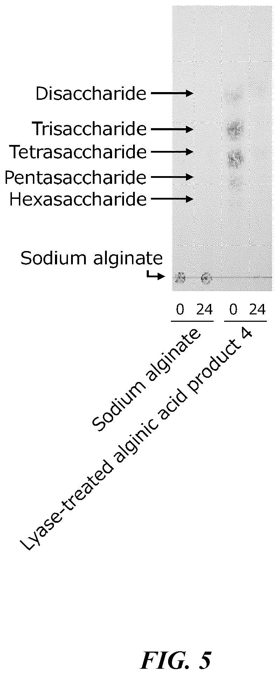

The TLC plate after coloration is shown in Fig. 5. It was observed that the spots corresponding to oligosaccharides having a polymerization degree of 2 to 6 became lighter or disappeared after the culture in the medium containing the lyase-treated alginic acid product 4. This shows that *Faecalibacterium prausnitzii* assimilated especially oligosaccharides having a polymerization degree of 2 to 6 of the lyase-treated alginic acid product 4.

### <Test Example 4> Examination of Degradation Degrees by Hydrolysis Treatment and Alginate Lyase Treatment of Sodium Alginate

### (1) Sample Preparation

The same reagents as those of Test Example 1 were used unless otherwise specified.

A commercial sodium alginate material in an amount of 3 g was dissolved with 99.7 mL of MilliQ water, and a sodium alginate solution was thus prepared. Immediately after adding 0.3 mL of acetic acid to the sodium alginate solution, a 4N NaOH solution was added to adjust the pH to 6.2±0.1, and an acetic acid-containing sodium alginate solution (sample a') was thus obtained.

To 100 mL of the acetic acid-containing sodium alginate solution (sample a'), 1 mL of an aqueous solution of 6 mg/mL alginate lyase S (manufactured by Nagase ChemteX Corporation) was added, and enzymatic reaction was advanced at 40°C for three hours. Next, the enzyme was inactivated by heating at 80°C for 30 minutes, and a lyase-treated alginic acid solution (sample b') was thus obtained.

Acetic acid was added to the lyase-treated alginic acid solution (sample b') to adjust the pH to 4.2±0.1, and autoclave treatment was conducted at 104°C for 7.5 hours. Then, a 4N NaOH solution was added to adjust the pH to 6.2±0.1, and a final sample of a hydrolysate solution of the lyase-treated alginic acid product (sample c) was thus obtained.

To the sodium alginate solution prepared above, 0.3 mL of acetic acid was added to adjust the pH to 4.2±0.1, and the mixture was subjected to autoclave treatment at 104°C for 7.5 hours. An alginate hydrolysate solution (sample d') was thus obtained.

A 4N NaOH solution was added to the alginate hydrolysate solution (sample d') to adjust the pH to 6.2±0.1. By the same procedures as those for preparing the sample b', lyase treatment reaction of the alginate hydrolysate solution (sample d') was conducted, and a lyase-treated alginate hydrolysate solution (sample e') was thus obtained.

Acetic acid was added to the acetic acid-containing sodium alginate solution (sample a'), the lyase-treated alginic acid solution (sample b'), the alginate hydrolysate solution (sample d') and the lyase-treated alginate hydrolysate solution (sample e'), and a 4N NaOH solution was added immediately to adjust the pH to 6.2±0.1. Final samples (samples a, b, d and e) were thus obtained. The treatments conducted for obtaining the samples are shown in Table 1.

### [Table 1]

**Table 1**

| | Enzymatic (Lyase) Degradation | Hydrolysis |
|---|---|---|
| Sample a | - | - |
| Sample b | + | - |
| Sample c | + | + (after enzymatic degradation) |
| Sample d | - | + |
| Sample e | + | + (before enzymatic degradation) |

### (2) Analysis of Degradation Degrees of Samples

The samples a to e were analyzed by thin-layer chromatography (TLC).

The samples obtained in (1) were diluted with MilliQ water, and thus obtained 3% (w/v) diluted solutions were used as TLC samples. Moreover, a 1% (w/v) solution of the lyase-treated alginic acid product 1 used in Test Example 1 was used as sample M.

The TLC was conducted by the same procedures as those of Test Example 2.

### (3) Results

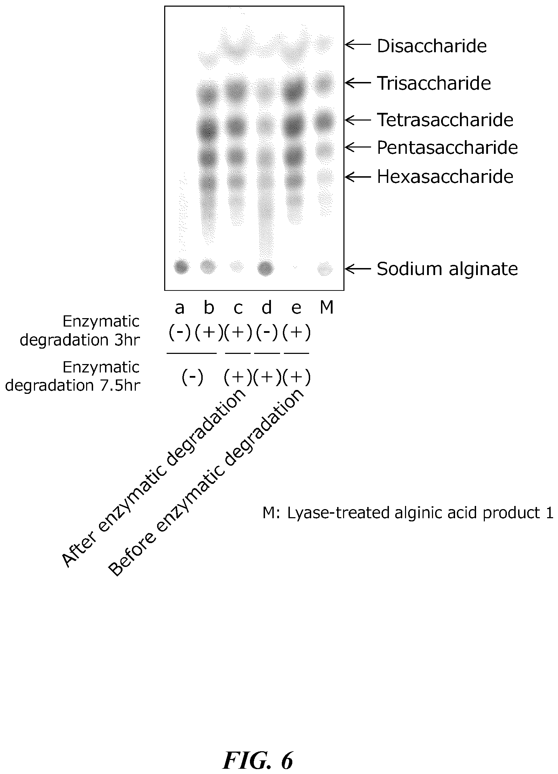

The TLC plate after coloration is shown in Fig. 6. It was found that the degradation degree increases and that oligosaccharides having a low polymerization degree are easily obtained when sodium alginate is hydrolyzed before or after conducting the enzymatic degradation.

### <Test Example 5> Single Culture 4 of Faecalibacterium prausnitzii

### (1) Medium Preparation

MilliQ water in a volume to be 80% of a specified volume was added to powder having YCFA medium composition and mixed, and the mixture was subjected to autoclave treatment at 115°C for 15 minutes. A YCFA medium preparation solution was thus obtained. The solutions of the samples a to e obtained in Test Example 4 were subjected to autoclave treatment at 115°C for 15 minutes. To the YCFA medium preparation solution, 1/4 the volume of the samples a to e after the autoclave treatment were added, and 0.6 mass% saccharide-containing YCFA media were thus prepared.

A commercial guar gum hydrolysate ("Sunfiber" (manufactured by Taiyo Kagaku Corporation)) was added to MilliQ water, and a specified amount of powder having YCFA medium composition excluding the saccharides was further added. Autoclave treatment was conducted at 115°C for 15 minutes, and a 0.6 mass% guar gum hydrolysate-containing medium was thus obtained.

A specified amount of powder having YCFA medium composition excluding the saccharides was added to 64.7 mL of MilliQ water. Autoclave treatment was conducted at 115°C for 15 minutes, and a negative control medium was thus obtained.

### (2) Single Culture

*Faecalibacterium prausnitzii* MCC2041 was cultured using the YCFA media prepared in (1) in the same manner as that in Test Example 1.

### (3) Analysis of Faecalibacterium prausnitzii-Assimilated Fractions

The polymerization degrees of the oligosaccharides assimilated by *Faecalibacterium prausnitzii* were analyzed by TLC in the same manner as that in Test Example 2.

The turbidities at a wavelength of 600 nm of the culture solutions were measured in the same manner as that in Test Example 1.

### (4) Results

From the results of TLC, it was observed that the spots corresponding to di-to hexaoligosaccharides became lighter after the culture in the sample b- to e-containing media. This shows that *Faecalibacterium prausnitzii* assimilated especially oligosaccharides having a polymerization degree of 2 to 6 of the lyase-treated alginic acid products.

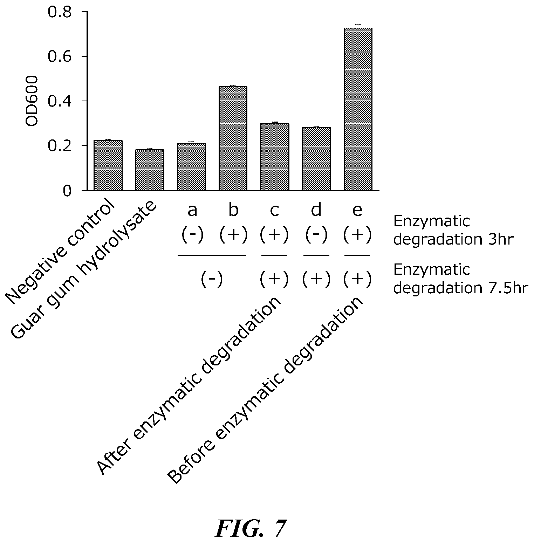

The turbidities (OD₆₀₀) of the culture solutions after 24 hours of the culture are shown in Fig. 7. *Faecalibacterium prausnitzii* could not assimilate sodium alginate (sample a) and the guar gum hydrolysate and hardly grew. On the other hand, growth of *Faecalibacterium prausnitzii* was observed in the lyase-treated alginic acid product (sample b). Moreover, significant growth of *Faecalibacterium prausnitzii* was observed in the lyase-treated alginate hydrolysate (sample e), which was obtained by hydrolyzing sodium alginate and then treating with the lyase.

In the hydrolysate of the lyase-treated alginic acid product (sample c), which was obtained by treating sodium alginate with the lyase and then hydrolyzing, the growth of *Faecalibacterium prausnitzii* slowed down even though the amounts of oligosaccharides having a low polymerization degree were higher than those of the sample b (see Fig. 6). This is believed to be because saturated forms of oligosaccharides were formed through the hydrolysis after the lyase treatment and because the amounts of oligosaccharides having an unsaturated form at the non-reducing end, which are easily assimilated by *Faecalibacterium prausnitzii,* became relatively low.

### <Test Example 6> Examination of Degradation Degrees by Hydrolysis Treatment and Alginate Lyase Treatment of Sodium Alginate

### (1) Sample Preparation

A commercial sodium alginate material ("I-5 (molecular weight of about 2.8 million)" (manufactured by KIMICA Corporation)) was dissolved in MilliQ water kept at 50°C, and a 3 mass% aqueous sodium alginate solution was thus prepared. After adding acetic acid (Kokusan Chemical Co., Ltd.) at 0.3 mass% to adjust the pH to 4.2±0.1, the mixture was subjected to autoclave treatment at 104°C for 3.5 hours or 7.5 hours, and alginate hydrolysate solutions were thus obtained. After further adding a 4N NaOH solution to adjust the pH to 6.2±0.1, alginate lyase S (manufactured by Nagase ChemteX Corporation) was added in an amount of 0.2 g per 100 g of the original sodium alginate material, and enzymatic reaction was advanced at 40°C for 0.5, 1, 2 or 3 hours. Next, the enzyme was inactivated by heating at 80°C for 30 minutes, and lyase-treated alginate hydrolysate solutions were thus obtained.

### (2) Analysis of Degradation Degrees of Samples

The lyase-treated alginate hydrolysate solutions were analyzed by thin-layer chromatography (TLC). The TLC was conducted by the same procedures as those of Test Example 2.

### (3) Results

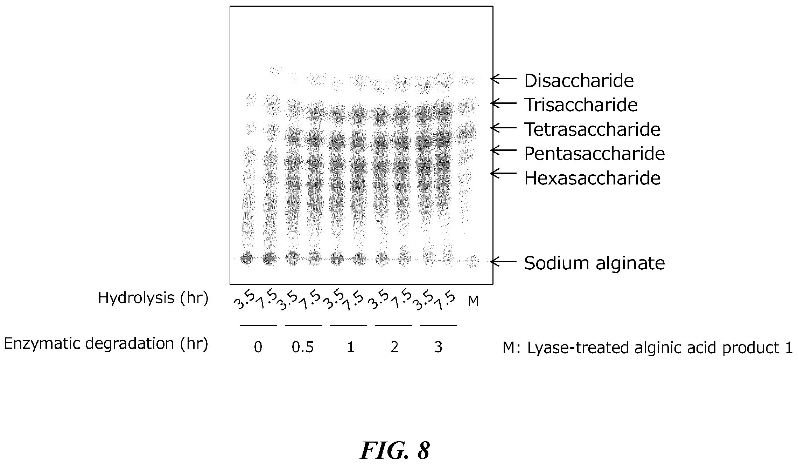

The TLC plate after coloration is shown in Fig. 8. It was found that the degradation degree increases and that oligosaccharides having a low polymerization degree are easily obtained when sodium alginate is hydrolyzed before the enzymatic degradation.

The aqueous solution of the sodium alginate material "I-5" used has a very high viscosity and is not suitable for directly subjecting to culture of *Faecalibacterium prausnitzii* or alginate lyase treatment. However, through hydrolysis treatment, degradation reaction by the alginate lyase is more easily conducted. As a result, the material is more easily assimilated by *Faecalibacterium prausnitzii* and can be a prebiotic which can promote the growth thereof.

### <Test Example 7> Fractionation of Lyase-Treated Alginic Acid Product by Nanofiltration Membrane (NF Membrane) Treatment

### (1) Acquisition and Fractionation of Alginate Oligosaccharide

A commercial potassium alginate material ("KULV-L3 (molecular weight of 40,000 to 60,000)" (manufactured by KIMICA Corporation)) in an amount of 400 g was dissolved using 7.6 L of water permeated through a reverse osmosis membrane (RO water). Alginate lyase S (manufactured by Nagase ChemteX Corporation) in an amount of 1.6 g was added thereto, and enzymatic reaction was advanced at 40°C for six hours. Next, the enzyme was inactivated by heating at 85°C for 10 minutes, and a lyase-treated potassium alginate product (also called "AOSK" below) was thus obtained. The obtained AOSK was diluted by adding 12 L of RO water, and 20 L of the diluted solution was permeated through an NF membrane (NTR7450, manufactured by Nitto Denko Corporation) under a pressure of 10 kgf/cm³ and thus fractionated. The NF membrane permeate solution was collected, and an NF membrane permeate solution of AOSK was thus obtained. The permeate solution was freeze-dried using a freeze dryer (type RL-B04), and 17.02 g of dry powder (also called "AOSK-NF" below) was thus obtained.

### (2) Comparison of Oligosaccharide Proportions before and after NF Membrane Treatment

The proportions of oligosaccharides having a polymerization degree of 2 to 6 contained in AOSK and AOSK-NF obtained in (1) were measured by HPLC. An aqueous AOSK solution and an aqueous AOSK-NF solution were prepared (each at 1 mass%), permeated through a filter of 0.22 µm (manufactured by Merck Millipore) and then subjected to HPLC. The system was Ultimate 3000 (manufactured by Thermo), and detection was conducted using a diode array detector (manufactured by Thermo). The column used was NH2P-50 4E (manufactured by Shoko Science Co., Ltd.). The mobile phase used was distilled water in which 0.3M sodium dihydrogen phosphate was dissolved, and isocratic measurement was made for 90 minutes. The flow rate was 1.0 mL/min, and the column oven temperature was set at 40°C. The detection was conducted by absorption at 235 nm. As the sample M, a 1% (w/v) solution of the lyase-treated alginic acid product 1 ("alginate oligosaccharide" (manufactured by Hokkaido Mitsui Chemicals, Inc.)) was used.

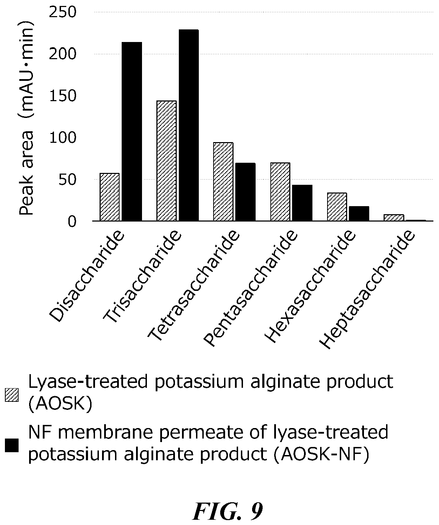

The peak areas in a HPLC chart of the oligosaccharides having corresponding polymerization degrees are shown in Fig. 9 as the oligosaccharide amounts contained in the lyase-treated potassium alginate product before and after the NF membrane treatment. It could be observed that, through the fractionation by the NF membrane treatment, the proportions of oligosaccharides having a polymerization degree of 2 or 3 in the oligosaccharides in the sample increased and that the amounts of oligosaccharides having a polymerization degree of 4 to 6 decreased.

### (3) Analysis of Polymerization Degrees of Oligosaccharides

The polymerization degrees of the oligosaccharides contained in AOSK and AOSK-NF obtained in (1) were analyzed by TLC.

An aqueous AOSK solution and an aqueous AOSK-NF solution were prepared (each at 1 mass%) and used for TLC analysis. The TLC was conducted by the same procedures as those of Test Example 2.

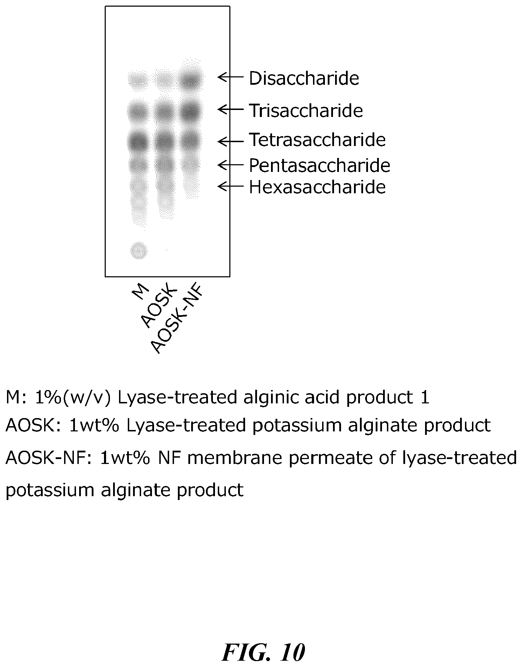

The TLC plate after coloration is shown in Fig. 10. The spots corresponding to oligosaccharides having a polymerization degree of 2 or 3 were darker in AOSK-NF than those in AOSK, and the spots corresponding to oligosaccharides having a polymerization degree of 4 or more were lighter. Thus, it was observed that, through the fractionation by the NF membrane treatment, the proportions of oligosaccharides having a polymerization degree of 2 or 3 in the oligosaccharides in the sample increased and that the amounts of tetrasaccharides to hexasaccharides decreased.

### <Test Example 8> Neutralizing Culture

### (1) Neutralizing Culture

A YCFA medium containing no saccharides in a volume of 100 mL was produced and put into vessels of a pH-controllable fermenter Bio Jr.8 (manufactured by Biott Corporation, BJR-25NA1S-8M), and 1 g of AOSK or AOSK-NF obtained in Test Example 7 was added. The media in the vessels were subjected to autoclave treatment at 115°C for 20 minutes. Then, a vitamin solution and a cysteine solution which were sterilized by filtration were aseptically added, and culture solutions containing the samples to be tested at a final concentration of 1% (w/v) were thus prepared. Then, the vessels were brought to anaerobic conditions through overnight nitrogen substitution, and 100 µL of fecal solutions which were adjusted to 10% (w/v) in advance with physiological saline were added. The solutions were cultured anaerobically for 68 hours at 37°C while the pH was controlled not to become 6 or less with a 1M Na₂CO₃ solution. The culture solutions in a volume of 1 mL were collected 0, 16, 20, 24, 48 and 68 hours after starting the culture.

The feces were provided by healthy humans (n=4; one male individual in the forties, two male individuals in the thirties and one male individual in the twenties) who had had a normal diet continuously.

### (2) Analysis of Enteric Bacteria

The culture solutions collected in (1) were centrifuged at 15,000g for 10 minutes, and the precipitates were obtained. The precipitates were suspended in 450 µL of an extraction solution (100 mM Tris/HCl, 4 mM EDTA, pH9.0) and then mixed with 50 µL of 10% SDS solution, 300 mg of glass beads having a diameter of 0.1 mm and 500 µL of TE saturated phenol (Wako Pure Chemical Industries, Ltd.), and the mixtures were subjected to pulverization treatment using FastPrep FP 100A (manufactured by Funakoshi Co., Ltd.) at power level 5 for 30 seconds. Next, after centrifugation at 14,000g for five minutes, 400 µL of the supernatants were taken, and 250 µL of phenol-chloroform solution (Wako Pure Chemical Industries, Ltd.) was added and mixed. After centrifugation at 14,000g for five minutes, 250 µL of the supernatants were obtained. The precipitates obtained by further adding 250 µL of 2-propanol were dissolved in 200 µL of Tris-EDTA buffer (pH8.0) and used as template DNA solutions.

Next, a 1st primer set for amplifying the third and fourth variable regions of 16S rRNA gene of bacteria (SEQ ID NOs: 1 and 2) and a 2nd primer set which is necessary for the analysis with a next-generation sequencer Miseq (manufactured by Illumina, Inc.) (SEQ ID NOs: 3 and 4, where n's are any nucleotide sequences for treating multiple samples in one analysis (index region)) were designed, and the primers were synthesized by the oligo primer production service of Life Technologies.

Reaction solutions containing the template DNA solutions and the 1st primer set having a total liquid volume of 25 µL were prepared using TaKaRa Ex Taq HS kit (manufactured by Takara Bio Inc.). PCR reaction of at 94°C for three minutes and then 20 cycles at 94°C for 30 seconds, at 50°C for 30 seconds and at 72°C for 30 seconds, followed by at 72°C for 10 minutes, was conducted using Veriti 200 (manufactured by Life Technologies). The obtained PCR products were subjected to electrophoresis with 1% agarose gel, and the band patterns were examined. Subsequently, using 1 µL of the obtained PCR products as templates, PCR was conducted under the same conditions as those described above using the 2nd primer set. The number of PCR cycles was 15. The obtained PCR products were subjected to electrophoresis with 1% agarose gel, and the band patterns were examined. Then, the PCR products were purified with QIAquick 96 PCR Purification Kit (manufactured by Qiagen), and the concentrations were measured with Quant-iT PicoGreen dsDNA Assay kit (manufactured by Life Technologies). Mixtures with the DNA solutions at the same concentrations were subjected to Miseq v2 Reagent kit (manufactured by Illumina, Inc.), and the sequences were analyzed by Miseq.

The compositions of the intestinal microbiota were analyzed by QIIME software (version 2.0) (http://qiime.org/) using the obtained paired-end sequences. The proportions of the dominant butyrate-producing bacterium (*Faecalibacterium prausnitzii*) in all the enteric bacteria were calculated, and the averages of all the samples to be tested were determined.

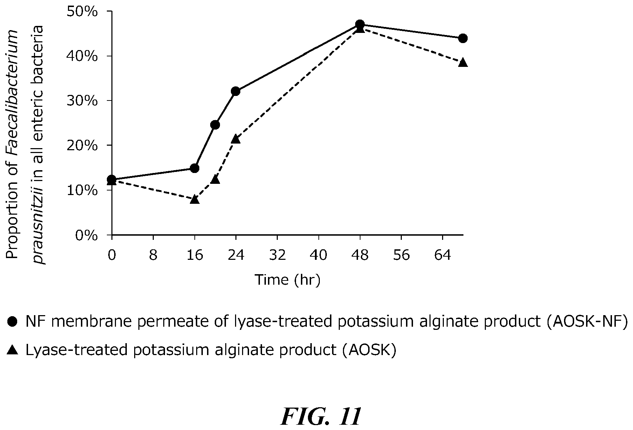

The changes with time of the proportion of *Faecalibacterium prausnitzii* in all the enteric bacteria are shown in Fig. 11. It was observed that the growth of *Faecalibacterium prausnitzii* was promoted and that the proportion in all the enteric bacteria increased because the proportions of oligosaccharides having a polymerization degree of 2 or 3 in the oligosaccharides in the sample were increased by the fractionation by the NF membrane treatment.

### <Test Example 9> Single Culture 5 of Faecalibacterium prausnitzii

### (1) Medium Preparation

MilliQ water in a volume adjusted to 80% of a specified volume was added to powder having YCFA medium composition and mixed, and the mixture was subjected to autoclave treatment at 115°C for 15 minutes. A YCFA medium preparation solution was thus obtained. AOSK or AOSK-NF obtained in Test Example 6 was added to MilliQ water, and aqueous 3% (w/v) oligosaccharide solutions were thus prepared and subjected to autoclave treatment at 115°C for 15 minutes. The aqueous oligosaccharide-containing solutions in 1/4 the volume were added to the YCFA medium preparation solution, and 0.6 mass% saccharide-containing YCFA media were thus prepared. Moreover, a specified amount of powder having YCFA medium composition excluding the saccharides was added to 64.7 mL of MilliQ water. The mixture was subjected to autoclave treatment at 115°C for 15 minutes, and a negative control medium was thus obtained.

### (2) Single Culture

*Faecalibacterium prausnitzii* MCC2041 was anaerobically cultured using the YCFA media prepared in (1) in the same manner as that in Test Example 1.

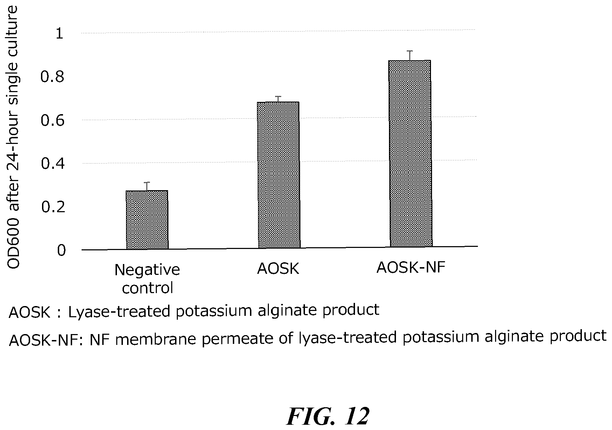

The turbidities (OD₆₀₀) of the culture solutions after 24 hours of the culture are shown in Fig. 12. It can be seen that the growth of *Faecalibacterium prausnitzii* was promoted more by AOSK-NF than AOSK.

### DRAWINGS

[Fig. 1] NEGATIVE CONTROL, POSITIVE CONTROL, NA ALGINATE, ALGINATE HYDROLYSATE, LYASE-TREATED ALGINIC ACID PRODUCT 1
[Fig. 2] NEGATIVE CONTROL, POSITIVE CONTROL, GUAR GUM HYDROLYSATE, NA ALGINATE, LYASE-TREATED ALGINIC ACID PRODUCT 1, LYASE-TREATED ALGINIC ACID PRODUCT 2, LYASE-TREATED ALGINIC ACID PRODUCT 3
[Fig. 3] DISACCHARIDE, TRISACCHARIDE, TETRASACCHARIDE, PENTASACCHARIDE, HEXASACCHARIDE, NA ALGINATE SODIUM ALGINATE, LYASE-TREATED ALGINIC ACID PRODUCT 1, LYASE-TREATED ALGINIC ACID PRODUCT 2, LYASE-TREATED ALGINIC ACID PRODUCT 3
[Fig. 4] NEGATIVE CONTROL, POSITIVE CONTROL, GUAR GUM HYDROLYSATE, NA ALGINATE, LYASE-TREATED ALGINIC ACID PRODUCT 4
[Fig. 5] DISACCHARIDE, TRISACCHARIDE, TETRASACCHARIDE, PENTASACCHARIDE, HEXASACCHARIDE, NA ALGINATE SODIUM ALGINATE, LYASE-TREATED ALGINIC ACID PRODUCT 4
[Fig. 6] DISACCHARIDE, TRISACCHARIDE, TETRASACCHARIDE, PENTASACCHARIDE, HEXASACCHARIDE, NA ALGINATE ENZYMATIC DEGRADATION 3 HR, HYDROLYSIS 7.5 HR AFTER ENZYMATIC DEGRADATION, BEFORE ENZYMATIC DEGRADATION M: LYASE-TREATED ALGINIC ACID PRODUCT 1
[Fig. 7] NEGATIVE CONTROL, GUAR GUM HYDROLYSATE, AFTER ENZYMATIC DEGRADATION, BEFORE ENZYMATIC DEGRADATION ENZYMATIC DEGRADATION 3 HR, HYDROLYSIS 7.5 HR
[Fig. 8] DISACCHARIDE, TRISACCHARIDE, TETRASACCHARIDE, PENTASACCHARIDE, HEXASACCHARIDE, NA ALGINATE HYDROLYSIS (HR), ENZYMATIC DEGRADATION (HR) M: LYASE-TREATED ALGINIC ACID PRODUCT 1
[Fig. 9] PEAK AREA (MAU·MIN) DISACCHARIDE, TRISACCHARIDE, TETRASACCHARIDE, PENTASACCHARIDE, HEXASACCHARIDE, HEPTASACCHARIDE LYASE-TREATED K ALGINATE PRODUCT (AOSK) NF MEMBRANE PERMEATE OF LYASE-TREATED K ALGINATE PRODUCT (AOSK-NF)
[Fig. 10] DISACCHARIDE, TRISACCHARIDE, TETRASACCHARIDE, PENTASACCHARIDE, HEXASACCHARIDE
   M: 1% (W/V) LYASE-TREATED ALGINIC ACID PRODUCT 1
   AOSK: 1 WT% LYASE-TREATED K ALGINATE PRODUCT
   AOSK-NF: 1 WT% NF MEMBRANE PERMEATE OF LYASE-TREATED K ALGINATE PRODUCT
[Fig. 11] PROPORTION OF *FAECALIBACTERIUM PRAUSNITZII* IN ALL ENTERIC BACTERIA
   TIME (HR)
   NF MEMBRANE PERMEATE OF LYASE-TREATED POTASSIUM ALGINATE PRODUCT (AOSK-NF)
   LYASE-TREATED POTASSIUM ALGINATE PRODUCT (AOSK)
[Fig. 12] OD600 AFTER 24-HOUR SINGLE CULTURE
   NEGATIVE CONTROL, LYASE-TREATED POTASSIUM ALGINATE PRODUCT (AOSK), NF MEMBRANE PERMEATE OF LYASE-TREATED POTASSIUM ALGINATE PRODUCT (AOSK-NF)

## Claims

1. A prebiotic composition for a butyrate-producing bacterium containing an oligosaccharide composed of β-D-mannuronic acid and/or α-L-guluronic acid or a salt thereof,
wherein the oligosaccharide has an unsaturated form of β-D-mannuronic acid residue or α-L-guluronic acid residue at the non-reducing end.

2. The composition according to claim 1, wherein the unsaturated form has a double bond between the carbon atoms at position 4 and position 5.

3. The composition according to claim 1 or 2, wherein the oligosaccharide contains an oligosaccharide having a polymerization degree of 2 to 10.

4. The composition according to any one of claims 1 to 3, wherein the butyrate-producing bacterium is a bacterium of *Faecalibacterium.*

5. The composition according to claim 4, wherein the bacterium of *Faecalibacterium* is *Faecalibacterium prausnitzii.*

6. A composition containing an oligosaccharide composed of β-D-mannuronic acid and/or α-L-guluronic acid or a salt thereof,
wherein the oligosaccharide has an unsaturated form of β-D-mannuronic acid residue or α-L-guluronic acid residue at the non-reducing end,
and the composition is administered or inoculated to a subject having a disease or a pathological condition which can be prevented or improved through an increase in butyric acid in the body or a subject having a disease or a pathological condition which is caused by a decrease in butyric acid in the body.

7. The composition according to any one of claims 1 to 6 which is used for intestinal regulation, immunomodulation, reduction in oxidative stress, prevention or improvement of diarrhea, prevention or improvement of an inflammatory bowel disease or prevention of large intestine cancer.

8. The composition according to any one of claims 1 to 7 which is a food or a drink.

9. The composition according to any one of claims 1 to 7 which is a pharmaceutical product.

10. Use of an oligosaccharide composed of β-D-mannuronic acid and/or α-L-guluronic acid or a salt thereof in the manufacture of a prebiotic composition for a butyrate-producing bacterium,
wherein the oligosaccharide has an unsaturated form of β-D-mannuronic acid residue or α-L-guluronic acid residue at the non-reducing end.

11. Use of an oligosaccharide composed of β-D-mannuronic acid and/or α-L-guluronic acid or a salt thereof in the promotion of the growth of a butyrate-producing bacterium,
wherein the oligosaccharide has an unsaturated form of β-D-mannuronic acid residue or α-L-guluronic acid residue at the non-reducing end.

12. An oligosaccharide which is composed of β-D-mannuronic acid and/or α-L-guluronic acid and which has an unsaturated form of β-D-mannuronic acid residue or α-L-guluronic acid residue at the non-reducing end or a salt thereof for use in the promotion of the growth of a butyrate-producing bacterium.

13. A method for promoting the growth of a butyrate-producing bacterium, including
administering an oligosaccharide composed of β-D-mannuronic acid and/or α-L-guluronic acid or a salt thereof to an animal,
wherein the oligosaccharide has an unsaturated form of β-D-mannuronic acid residue or α-L-guluronic acid residue at the non-reducing end.

14. A method for producing a prebiotic composition for a butyrate-producing bacterium, including a step of causing an alginate lyase to act on alginic acid and/or a salt thereof or a hydrolysate thereof and thus obtaining a degradation product of alginic acid.

15. The method according to claim 14, wherein the alginate lyase is an endo-type alginate lyase and/or an exo-type alginate lyase.

16. The method according to claim 14 or 15 which includes a step of hydrolyzing the alginic acid and/or the salt thereof and thus obtaining an alginate hydrolysate and a step of causing the alginate lyase to act on the alginate hydrolysate and thus obtaining the degradation product of alginic acid.

17. The method according to any one of claims 14 to 16 which further includes a step of collecting an oligosaccharide having a polymerization degree of 2 to 3 and/or a salt thereof.

18. The method according to any one of claims 14 to 17, wherein the butyrate-producing bacterium is a bacterium of *Faecalibacterium.*

19. The method according to claim 18, wherein the bacterium of *Faecalibacterium* is *Faecalibacterium prausnitzii.*
